# EUROPEAN PATENT APPLICATION

(11) **EP 4 494 484 A1**
(43) Date of publication of application: **22.01.2025**
(21) Application number: 23770904.3
(22) Date of filing: 17.03.2023
(51) Int. Cl.: A23L 33/125, A23L 33/10, A23L 33/135, A61K 31/7004, A61K 31/7008, A61K 31/7012, A61K 31/7016, A61K 31/702, A61K 35/20, A61P 1/00, A61P 1/04, A61P 1/12, A61P 1/14, A61P 1/16, A61P 3/00, A61P 3/04, A61P 9/06, A61P 11/06, A61P 13/12, A61P 17/00, A61P 25/00

(54) **COLLINSELLA BACTERIA PROLIFERATION CONTROLLING COMPOSITION AND USE THEREOF**

(30) Priority: 18.03.2022 JP 2022044397; 18.03.2022 JP 2022044398; 18.03.2022 JP 2022044399; 18.03.2022 JP 2022044400; 18.03.2022 JP 2022044401; 18.03.2022 JP 2022044402; 18.03.2022 JP 2022044403; 18.03.2022 JP 2022044404; 18.03.2022 JP 2022044405; 18.03.2022 JP 2022044406; 18.03.2022 JP 2022044407
(71) Applicant: MEIJI CO., LTD, Chuo-ku Tokyo 104-8306 (JP)
(72) Inventor: FUJIWARA, Shin, Hachioji-shi, Tokyo 192-0919 (JP); TAKEDA, Mariko, Hachioji-shi, Tokyo 192-0919 (JP)
(74) Representative: TBK
(86) International application number: PCT/JP2023/010497
(87) International publication number: WO 2023/176951

(57) **Abstract**

A means for controlling growth of bacteria of the genus *Collinsella* in the intestinal tract is provided. An object of the present invention is to provide a novel material that can control growth of bacteria of the genus *Collinsella.* There is provided a composition for controlling growth of bacteria of the genus *Collinsella* in intestinal bacterial microbiota, which contains any selected from the group consisting of a human milk oligosaccharide and a constituent sugar of a human milk oligosaccharide.

## Description

### Technical Field

The present invention relates to a composition for controlling growth of bacteria of the genus *Collinsella* and use thereof.

### Background Art

Bacteria of the genus *Collinsella* are intestinal bacteria present in the human intestines, and it is known that, especially among them, *C. aerofaciens* is abundant in the intestines of responders to anti-PD-1 antibodies found in cancer immunotherapies (Non-patent document 1), and has a key gene relating to the effectiveness of the fecal transplantation therapy for recurrent *Clostridium difficile* infection (Non-patent document 2). Recently, it has also been reported that a machine learning model (generalized linear model, GLM) was developed to predict COVID-19 mortality rate in each country using the intestinal microbiota, and the results of the GLM analysis showed that the lower the proportion of bacteria of the genus *Collinsella* in the intestinal microbiota, the higher the COVID-19 mortality rate (Non-patent document 3).

With regard to bacteria of the genus *Collinsella,* WholeFiber and inulin are known as materials that allow growth of bacteria of the genus *Collinsella* in the intestines (Non-patent document 4). Patent document 1 describes a method for adsorbing bile acids characterized by using intestinal bacteria that have a property of taking up bile acids into the bacterial cells in the presence of a sugar source, and mentions bacteria of the genus *Collinsella* as one class of the intestinal bacteria to be used. Further, Patent document 2 describes a composition containing at least one bacterial strain having a bile acid deconjugating activity and/or 7-epimerization activity for use in methods for treating intrahepatic bile stasis in a subject or reducing the risk of the same, and mentions *Collinsella aerofaciens,* ATCC25986 as one of the bacterial strains to be contained in the composition. Furthermore, Patent document 3 describes a method for producing L-ornithine and equol, comprising (1) the step of culturing an anaerobic microorganism in a medium containing isoflavones and dextrin, and (2) the step of collecting L-ornithine and equol obtained in the step (1), and mentions bacteria of the genus *Collinsella* as one class of the anaerobic microorganism. Patent document 4 describes a composition capable of efficiently treating and preventing inflammation, especially ulcerative colitis, characterized in that it contains *Megamonas funiformis* and/or metabolites thereof and *Anaerofustis stercorihominis* and/or metabolites thereof, and describes that the composition further contains *Collinsella shenzhenensis* and/or metabolites thereof, and *Collinsella shenzhenensis* is preferably *Collinsella shenzhenensis* TF06-26, of which deposition number is GDMCC60090. Patent document 5 describes a human intestinal bacterial microbiota model for evaluating a short-chain fatty acids increase promoting action and/or a diversity increase promoting action in a human intestine of a target food composition, which is constituted with a bacterial culture for evaluation containing at least 9 kinds of bacterial species including bacteria belonging to the genus *Bifidobacterium,* but not containing bacteria belonging to the phylum *Proteobacteria* or the genus *Lactobacillus,* and mentions bacteria of the genus *Collinsella* as one class of the bacteria that can be contained in that model.

By the way, human milk oligosaccharides (HMOs) are the third most abundant solid component in breast milk after lactose and lipids. It is known that feeding 2'-fucosyllactose, 3-fucosyllactose, and lactodifucotetraose, which are types of human milk oligosaccharides, causes bifidobacteria to grow (Non-patent document 5).

### Prior Art References

### Patent documents

Patent document 1: Japanese Patent Publication (Kokai) No. 2006-314219
Patent document 2: WO2017/102816 (Japanese Patent Publication (Kohyo) No. 2018-537484)
Patent document 3: Japanese Patent Publication (Kokai) No. 2014-54234
Patent document 4: WO2019/227418 (Japanese Patent Publication (Kohyo) No. 2021-524751)
Patent document 5: WO2021/206106

### Non-patent documents

Non-patent document 1: Matson V, Fessler J, Bao R, et al. The commensal microbiome is associated with anti-PD-1 efficacy in metastatic melanoma patients. Science. 2018;359(6371):104-108. doi:10.1126/science.aao3290
Non-patent literature 2: Mullish BH, McDonald JAK, Pechlivanis A, et al. Microbial bile salt hydrolases mediate the efficacy of faecal microbiota transplant in the treatment of recurrent Clostridioides difficile infection. Gut. 2019;68(10):1791-1800. doi:10.1136/gutjnl-2018-317842
Non-patent literature 3: Hirayamal M, Nishiwaki H, et al. Intestinal Collinsella may mitigate infection and exacerbation of COVID-19 by producing ursodeoxycholate. PLoS One. 2021 Nov 23;16(11):e0260451. doi: 10.1371/journal.pone.0260451. eCollection 2021
Non-patent document 4: Carlson JL, Erickson JM, Hess JM, Gould TJ, Slavin JL. Prebiotic Dietary Fiber and Gut Health: Comparing the in Vitro Fermentations of Beta-Glucan, Inulin and Xylooligosaccharide. Nutrients. 2017;9(12):1361. Published 2017 Dec 15. doi:10.3390/nu9121361
Non-patent document 5: Yu ZT, Chen C, Newburg DS. Utilization of major fucosylated and sialylated human milk oligosaccharides by isolated human gut microbes. Glycobiology. 2013;23(11):1281-1292. doi:10.1093/glycob/cwt065

### Summary of the Invention

### Object to be achieved by the invention

Bacteria of the genus *Collinsella* are considered to be one of the useful intestinal bacteria. It would be desirable if there were novel materials that can promote growth of bacteria of the genus *Collinsella* in the intestinal tract.

### Means for achieving the object

The present invention provides the followings.
[1] A composition for controlling growth of bacteria of the genus *Collinsella* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid.
[3] The composition according to 1 or 2, which contains any selected from the group consisting of 2'-fucosyllactose, 3'-sialyllactose, 6'-sialyllactose, lactose, and N-acetylneuraminic acid.
[4] The composition according to any one of 1 to 3, which is for use as a prebiotic or synbiotic.
[5] A composition for treating a disease or condition that can be ameliorated by controlling growth of bacteria of the genus *Collinsella* in the intestinal tract, which contains any selected from the group consisting of a human milk oligosaccharide and a constituent sugar of a human milk oligosaccharide.
[6] The composition according to 5, wherein the disease or condition that can be ameliorated by controlling growth of bacteria of the genus *Collinsella* in the intestinal tract is a respiratory tract infection.
[7] A composition for treating a respiratory tract infection, which contains any selected from the group consisting of a human milk oligosaccharide and a constituent sugar of a human milk oligosaccharide.
[8] The composition according to 6 or 7, wherein the respiratory tract infection is a coronavirus infection.
[9] The composition according to 6 or 7, wherein the respiratory tract infection is a novel coronavirus infection (COVID-19).
[10] The composition according to any one of 1 to 5, wherein the control of growth is promotion of growth.
[11] A composition for use in a method for controlling growth of bacteria of the genus *Collinsella* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Collinsella* in the intestinal bacterial microbiota, a method or non-therapeutic method for controlling growth of bacteria of the genus *Collinsella* in the intestinal bacterial microbiota, which comprises the step of administering a composition containing any selected from the group consisting of a human milk oligosaccharide and a constituent sugar of a human milk oligosaccharide to a subject, or use or non-therapeutic use of a composition containing any selected from the group consisting of a human milk oligosaccharide and a constituent sugar of a human milk oligosaccharide for controlling growth of bacteria of the genus *Collinsella* in the intestinal bacterial microbiota.
[12] The composition according to 11, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid; the use in manufacture according to 11, which is of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid; the method or non-therapeutic method according to 11, which comprises the step of administering a composition containing any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid to a subject; or the use or non-therapeutic use according to 11, which is of such a composition.
[13] The composition according to 7 or 8, which contains any selected from the group consisting of 2'-fucosyllactose, 3'-sialyllactose, 6'-sialyllactose, lactose, and N-acetylneuraminic acid; the use in manufacture according to 11 or 12, which is of any selected from the group consisting of 2'-fucosyllactose, 3'-sialyllactose, 6'-sialyllactose, lactose, and N-acetylneuraminic acid; the method or non-therapeutic method according to 11 or 12, which comprises the step of administering a composition containing any selected from the group consisting of 2'-fucosyllactose, 3'-sialyllactose, 6'-sialyllactose, lactose, and N-acetylneuraminic acid to a subject; or the use or non-therapeutic use according to 11 or 12, which is of such a composition.
[14] The composition, use in manufacture, method or non-therapeutic method, or use or non-therapeutic use according to any one of 11 to 13, wherein the composition is for use as a prebiotic or synbiotic.
[15] A composition for use in a method for treating a disease or condition that can be ameliorated by controlling growth of bacteria of the genus *Collinsella* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Collinsella* in the intestinal tract, a method or non-therapeutic method for treating a disease or condition that can be ameliorated by controlling growth of bacteria of the genus *Collinsella* in the intestinal tract, which comprises the step of administering a composition containing any selected from the group consisting of a human milk oligosaccharide and a constituent sugar of a human milk oligosaccharide to a subject, or use or non-therapeutic use of a composition containing any selected from the group consisting of a human milk oligosaccharide and a constituent sugar of a human milk oligosaccharide for treating a disease or condition that can be ameliorated by controlling growth of bacteria of the genus *Collinsella* in the intestinal tract.
[16] The composition, use in manufacture of a composition, method or non-therapeutic method, or use or non-therapeutic use according to 15, wherein the disease or condition that can be ameliorated by controlling of growth of bacteria of the genus *Collinsella* in the intestinal tract is a respiratory tract infection.
[17] A composition for treating a respiratory tract infection, which contains any selected from the group consisting of a human milk oligosaccharide and a constituent sugar of a human milk oligosaccharide, a composition for use in a method for treating a respiratory tract infection, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a respiratory tract infection, a method or non-therapeutic method for treating a respiratory tract infection, which comprises the step of administering a composition containing any selected from the group consisting of a human milk oligosaccharide and a constituent sugar of a human milk oligosaccharide to a subject, or use or non-therapeutic use of a composition containing any selected from the group consisting of a human milk oligosaccharide and a constituent sugar of a human milk oligosaccharide for treating a respiratory tract infection.
[18] The composition, use in manufacture of a composition, method or non-therapeutic method, or use or non-therapeutic use according to 16 or 17, wherein the respiratory tract infection is a coronavirus infection.
[19] The composition, use in manufacture of a composition, method or non-therapeutic method, or use or non-therapeutic use according to any one of 16 to 18, wherein the respiratory tract infection is a novel coronavirus infection (COVID-19).
[20] The composition, use in manufacture of a composition, method or non-therapeutic method, or use or non-therapeutic use according to any one of 11 to 16, wherein the control of growth is promotion of growth.

### Effects of the Invention

According to the present invention, the intestinal bacterial microbiota can be controlled. In particular, increase of bacteria of the genus *Collinsella* in the intestinal bacterial microbiota can be controlled.

It can be expected that through controlling increase of bacteria of the genus *Collinsella* in the intestinal bacterial microbiota, various diseases or conditions that can be ameliorated by such a control, for example, respiratory tract infections such as COVID-19 infection, can be treated.

By adding the active ingredients of the present invention, functional foods can be provided in various forms.

### Brief Description of the Drawing

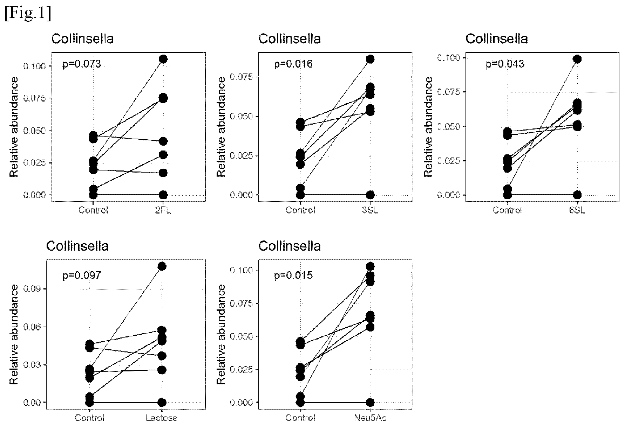

[Fig. 1] Changes in occupancy rate of bacteria of the genus *Collinsella* induced by various carbohydrates in a human fecal fermentation system, evaluated by the paired t-test.

### Modes for Carrying out the Invention

Hereafter, the present invention will be explained in detail.

The present invention relates to a composition for controlling growth of bacteria of the genus *Collinsella,* which contains any selected from the group consisting of a human milk oligosaccharide (also referred to as HMO) and a constituent sugar of a human milk oligosaccharide as an active ingredient.

### [Active Ingredient]

The composition of the present invention contains any selected from the group consisting of a human milk oligosaccharide (also referred to as HMO) and a constituent sugar of a human milk oligosaccharide as an active ingredient. Human milk oligosaccharides are oligosaccharides contained in human milk and are the third most abundant solid component after lactose and lipids. With few exceptions, human milk oligosaccharides have a chemical structure comprising a lactose unit at the reducing end, to which fucose, sialic acid, galactose and so forth are added.

For the present invention, the abbreviation and structure of milk oligosaccharides are according to the desc Nutr Rev. 2017 Nov 1;75(11):920-933. doi: 10.1093/nutrit/nux044; and Nutrients 2021, 13(8), 2737; https://doi.org/10.3390/nu13082737. In case of discrepancies between these references, the latter should be followed.

The human milk oligosaccharide and constituent sugar of a human milk oligosaccharide used in the composition are not particularly limited so long as they can provide the desired effect. The human milk oligosaccharide and constituent sugar of a human milk oligosaccharide used in the composition may consist of one type or a combination of two or more types thereof.

Examples of the human milk oligosaccharide contained in the composition include the following neutral and acidic oligosaccharides
Neutral oligosaccharides: 2'-fucosyllactose (2'-FL), 3-fucosyllactose (3-FL), difucosyllactose (DFL), lacto-difucotetraose (LDFT), lacto-N-tetraose (LNT), lacto-N-neotetraose (LNnT), lacto-N-fucopentaose I (LNFP I), lacto-N-fucopentaose II (LNFP II), lacto-N-fucopentaose III (LNFP III), lacto-N-fucopentaose V (LNFP V), lacto-N-fucopentaose VI (LNFP VI), lacto-N-difucohexaose I (LNDFH I), lacto-N-difucohexaose II (LNDFH II), lacto-N-hexaose (LNH), para-lacto-N-hexaose (pLNH), lacto-N-neohexaose (LNnH), para-lacto-N-neohexaose (pLNnH), fucosyllacto-N-hexaose I (F-LNH I), fucosyl-para-lacto-N-hexaose I (F-para-LNH I), fucosyllacto-N-hexaose II (F-LNH II), fucosyllacto-N-hexaose III (F-LNH III), difucosyllacto-N-hexaose I (DF-LNH I), difucosyllacto-N-hexaose II (DF-LNH II), difucosyllacto-N-hexaose III (DF-LNH III), difucosyl-para-lacto-N-neohexaose (DF -pLNnH), difucosyl-para-lacto-N-hexaose (DF-para-LNH), difucosyl-para-lacto-N-neohexaose (DF-para-LNnH), and trifucosyllacto-N-hexaose (TF-LNH).
Acidic oligosaccharides: 3'-sialyllactose (3'-SL), 6'-sialyllactose (6'-SL), 6'-sialyl-N-acetyllactosamine (6'-SLN), LS-tetrasaccharide a (LST a, sialyllacto-N-tetraose a), LS-tetrasaccharide b (LST b, sialyllacto-N-tetraose b), LS-tetrasaccharide c (LST c, sialyllacto-N-tetraose c), disialyllacto-N-tetraose (DS-LNT), fucosyl-sialyllacto-N-tetraose a (F-LST a), fucosyl-sialyllacto-N-tetraose b (F-LST b), fucosyl-sialyllacto-N-hexaose (FS-LNH), fucosyl-sialyllacto-N-neohexaose (FS-LNnH I), fucosyl-disialyllacto-N-hexaose (FDS-LNH), and disialyllacto-N-hexaose (DS-LNH).

Preferred examples of the human milk oligosaccharide contained in the composition include the following neutral and acidic oligosaccharides.
Neutral oligosaccharides: 2'-fucosyllactose (2'-FL), 3-fucosyllactose (3-FL), difucosyllactose (DFL), lacto-difucotetraose (LDFT), lacto-N-tetraose (LNT), lacto-N-neotetraose (LNnT), lacto-N-fucopentaose I (LNFP I), lacto-N-fucopentaose II (LNFP II), lacto-N-fucopentaose III (LNFP III), lacto-N-fucopentaose V (LNFP V), lacto-N-fucopentaose VI (LNFP VI), lacto-N-difucohexaose I (LNDFH I), lacto-N-difucohexaose II (LNDFH II), lacto-N-hexaose (LNH), lacto-N-neohexaose (LNnH), para-lacto-N-neohexaose (pLNnH), fucosyllacto-N-hexaose I (F-LNH I), fucosyl-para-lacto-N-hexaose I (F-para-LNH I), fucosyllacto-N-hexaose II (F-LNH II), fucosyllacto-N-hexaose III (F-LNH III), difucosyllacto-N-hexaose I (DF-LNH I), difucosyllacto-N-hexaose II (DF-LNH II), difucosyllacto-N-hexaose III (DF-LNH III), difucosyl-para-lacto-N-neohexaose (DF-pLNnH), and trifucosyllacto-N-hexaose (TF-LNH).
Acidic oligosaccharides: 3'-sialyllactose (3'-SL), 6'-sialyllactose (6'-SL), LS-tetrasaccharide a (LST a, sialyllacto-N-tetraose a), LS-tetrasaccharide b (LST b, sialyllacto-N-tetraose b), LS-tetrasaccharide c (LST c, sialyllacto-N-tetraose c), disialyllacto-N-tetraose (DS-LNT), fucosyl-sialyllacto-N-neohexaose (FS-LNnH I), and disialyllacto-N-hexaose (DS-LNH).

More preferred examples of the human milk oligosaccharide contained in the composition include the following neutral and acidic oligosaccharides.
Neutral oligosaccharides: 2'-fucosyllactose (2'-FL), difucosyllactose (DFL), lacto-difucotetraose (LDFT), lacto-N-tetraose (LNT), and lacto-N-neotetraose (LNnT). Acidic oligosaccharides: 3'-sialyllactose (3'-SL), and 6'-sialyllactose (6'-SL)

For the present invention, the term constituent sugar of a human milk oligosaccharide refers to a sugar constituting a human milk oligosaccharide (sugar resulting from degradation of human milk oligosaccharide), excluding glucose and galactose. Examples of the constituent sugar of a human milk oligosaccharide contained in the composition include lactose, fucose, sialic acid, and N-acetylglucosamine. Sialic acid is a general term for modifications of neuraminic acid. An example of sialic acid is N-acetylneuraminic acid (Neu5Ac), which is an acetyl-modified neuraminic acid.

According to the studies of the inventors of the present invention, there were confirmed superior growth-promoting effects of lactose, Neu5Ac, 2'-FL, 3'-SL, and 6'-SL for bacteria of the genus *Collinsella.* By the way, human milk oligosaccharides can generate lactose in the human digestive tract after ingestion. In addition, human milk oligosaccharides containing Neu5Ac as a constituent sugar can produce Neu5Ac in the human digestive tract after ingestion (see Non-patent documents 6 to 8 mentioned later). Furthermore, since the desired effect was confirmed for all of 2'-FL, 3'-SL, and 6'-SL, the desired effect would be sufficiently obtained with fucosylated human milk oligosaccharides and sialylated human milk oligosaccharides.

From this point of view, preferred examples of the human milk oligosaccharide and constituent sugar of a human milk oligosaccharide used in the composition are any selected from the group consisting of fucosylated human milk oligosaccharides, sialylated human milk oligosaccharides, a human milk oligosaccharide containing lactose as a constituent sugar, a human milk oligosaccharide containing sialic acid as a constituent sugar, lactose, and sialic acid. Human milk oligosaccharides containing sialic acid as a constituent sugar include sialylated human milk oligosaccharides (e.g., 3'-SL and 6'-SL) and human milk oligosaccharides containing sialic acid (e.g., LST a and DS-LNT).

Specific examples of the human milk oligosaccharide and constituent sugar of a human milk oligosaccharide used in the composition are any selected from the group consisting of 2'-FL, 3'-SL, 6'-SL, lactose, and Neu5Ac.

### [Use]

The composition of the present invention can be used for controlling growth of bacteria of the genus *Collinsella* in the intestinal bacterial microbiota. The control of growth includes promotion of growth and suppression of growth. Preferred compositions are for promotion of growth.

Examples of bacteria of the intestinal bacterial microbiota include the followings:
bacteria of the genus *Faecalibacterium,* e.g., *Faecalibacterium prausnitzii;*
bacteria of the genus *Akkermansia,* e.g., *Akkermansia muciniphila;*
bacteria of the genus *Blautia,* e.g., *Blautia coccoides, Blautia intestinalis,* and *Blautia faecicola;*
bacteria of the genus *Subdoligranulum,* e.g., *Subdoligranulum variabile;*
bacteria of the genus *Bilophila* e.g., *Bilophila wadsworthia;*
bacteria of the genus *Collinsella,* e.g., *Collinsella aerofaciens* and *Collinsella intestinalis;*
bacteria of the genus *Holdemania,* e.g., *Holdemania filiformis;*
bacteria of the genus *Parasutterella,* e.g., *Parasutterella excrementihominis* and *Parasutterella secunda;*
bacteria of the genus *Oscillibacter,* e.g., *Oscillibacter valericigenes;*
bacteria of the genus *Eggerthella,* e.g., *Eggerthella lenta;*
bacteria of the genus *Sutterella,* e.g., *Sutterella parvirubra* and *Sutterella wadsworthensis;* and
bacteria of the genus *Bifidobacterium* (bifidobacteria), e.g., *B. adolescentis, B. bifidum, B. breve, B. catenulatum, B. infantis, B. lactis, B. longum,* and *B. pseudocatenulatum.*

For the present invention, the term bacteria of the genus *Collinsella* refers to bacteria identified as belonging to the genus *Collinsella* by molecular phylogenetic analysis based on the 16S rRNA gene. Criteria for determining genus by molecular phylogenetic analysis based on the 16S rRNA gene are well known by those skilled in the art (Stackebrandt E, Ebers J. Taxonomic parameters revisited: tarnished gold standards. Microbiol Today. 2006;33:152-155).

For the present invention, controlling growth of specific bacteria in bacterial microbiota means controlling the proportion (occupancy rate) of the specific bacteria in the bacterial microbiota.

Whether or not a certain ingredient controls growth of a particular type of bacteria in the intestinal bacterial microbiota can be evaluated as follows. A fecal material provided by a healthy person is added to an appropriate medium, cultured for a certain period of time as required, then an ingredient to be evaluated is added, cultured under appropriate conditions (e.g., at 37°C under anaerobic conditions, similar to those in the intestinal tract, for 48 hours), and the occupancy rate of the particular type of bacteria in the bacterial microbiota in the culture is measured. The result of the measurement can then be compared with a result of a culture obtained under conditions that differ only in that a control (e.g., sterile water) is added in place of the ingredient to be evaluated but that are otherwise identical, and it can be determined that, if the occupancy rate is higher than the control, growth is promoted, and if lower, growth is suppressed.

Occupancy rate of a particular type of bacteria in bacterial microbiota can be determined by known methods. One preferred method is to perform 16S metagenomic analysis (sequence analysis of 16S rRNA gene amplicons) on DNAs extracted from culture. When the 16S metagenomic analysis is performed, DNA extraction can be performed by using commercially available kits. The genomic region to be analyzed is not particularly limited so long as the bacteria can be determined, but the V3-V4 region of the 16S rRNA gene can be used. Primers, amplification conditions, method for purification of amplicons, and so forth well known to those skilled in the art can be used to analyze bacteria. Sequence decoding is preferably performed on a next-generation sequencer of higher performance. A next-generation microbiome bioinformatics platform such as QIIME 2^{™} can be used to analyze the obtained data. For the 16S metagenomic analysis, those skilled in the art can refer to such information as Sanschagrin S, Yergeau E. Next-generation sequencing of 16S ribosomal RNA gene amplicons. J Vis Exp. 2014;(90):51709. Published 2014 Aug 29. doi: 10.3791/51709.

Bacteria of the genus *Collinsella* as the object of the present invention are intestinal bacteria present in the human intestines, among which C. *aerofaciens* is known to be abundant in the intestines of responders to anti-PD-1 antibodies found in cancer immunotherapies (Non-patent document 1 mentioned above), and has a key gene relating to the effectiveness of the fecal transplantation therapy for recurrent *Clostridium difficile* infection (Non-patent document 2 mentioned above). Therefore, the composition can be expected to be used for treating such a disease or condition.

The composition can also be used for treating a disease or condition that can be ameliorated by controlling growth of bacteria of the genus *Collinsella* in the intestinal tract. Such a disease or condition can be various diseases or conditions.

Recently, it has been reported that the lower the proportion of bacteria of the genus *Collinsella* in the intestinal bacterial microbiota of COVID-19 patients, the higher the mortality rate of the patients (Non-patent document 3). In addition, bacteria of the genus *Collinsella* are known to have genes involved in production of ursodeoxycholic acid, which is a secondary bile acid (Non-patent document 9), and ursodeoxycholic acid is known to prevent SARS-CoV-2 from binding to the receptor angiotensin converting enzyme 2 (ACE2), to which the virus binds first at the time of infection (Non-patent documents 10 and 11). Furthermore, ursodeoxycholic acid is known to suppress inflammation-inducing cytokines (Non-patent documents 12 and 13), have antioxidant and anti-apoptotic actions (Non-patent documents 14 and 15), and increase alveolar fluid clearance in acute respiratory distress syndrome (ARDS) (Non-patent document 16). Therefore, the composition that can control growth of bacteria of the genus *Collinsella* in the intestines can be used to treat respiratory tract infections such as COVID-19 infection.
Non-patent document 9: Liu L, Aigner A, Schmid RD. Identification, cloning, heterologous expression, and characterization of a NADPH-dependent 7β-hydroxysteroid dehydrogenase from Collinsella aerofaciens. Appl Microbiol Biotechnol. 2011;90(1):127-135. doi:10.1007/s00253-010-3052-y
Non-patent document 10: Poochi SP, Easwaran M, Balasubramanian B, Anbuselvam M, Meyyazhagan A, Park S, et al. Employing bioactive compounds derived from Ipomoea obscura (L.) to evaluate potential inhibitor for SARSCoV-2 main protease and ACE2 protein. Food Front. 2020. Epub 2020/08/25. https://doi.org/10.1002/fft2.29 PMID: 32838301; PubMed Central PMCID: PMC7361879
Non-patent document 11: Carino A, Moraca F, Fiorillo B, Marchiano S, Sepe V, Biagioli M, et al. Hijacking SARS-CoV-2/ACE2 Receptor Interaction by Natural and Semisynthetic Steroidal Agents Acting on Functional Pockets on the Receptor Binding Domain. Front Chem. 2020;8:572885. Epub 2020/11/17. doi: 10.3389/fchem.2020.572885; PubMed Central PMCID: PMC7645072
Non-patent document 12: Ko WK, Lee SH, Kim SJ, Jo MJ, Kumar H, Han IB, et al. Anti-inflammatory effects of ursodeoxycholic acid by lipopolysaccharide-stimulated inflammatory responses in RAW 264.7 macrophages. PLoS One. 2017;12(6):e0180673. Epub 2017/07/01. doi: 10.1371/journal.pone.0180673; PubMed Central PMCID: PMC5493427
Non-patent document 13: Ko WK, Kim SJ, Jo MJ, Choi H, Lee D, Kwon IK, et al. Ursodeoxycholic Acid Inhibits Inflammatory Responses and Promotes Functional Recovery After Spinal Cord Injury in Rats. Mol Neurobiol. 2019;56(1):267-77. Epub 2018/04/25. doi: 10.1007/s12035-018-0994-z
Non-patent document 14: Lapenna D, Ciofani G, Festi D, Neri M, Pierdomenico SD, Giamberardino MA, et al. Antioxidant properties of ursodeoxycholic acid. Biochem Pharmacol. 2002;64(11):1661-7. Epub 2002/11/14. doi: 10.1016/s0006-2952(02)01391-6
Non-patent document 15: Kim YJ, Jeong SH, Kim EK, Kim EJ, Cho JH. Ursodeoxycholic acid suppresses epithelial-mesenchymal transition and cancer stem cell formation by reducing the levels of peroxiredoxin II and reactive oxygen species in pancreatic cancer cells. Oncol Rep. 2017;38(6):3632-8. Epub 2017/11/14. doi: 10.3892/or.2017.6045
Non-patent document 16: Niu F, Xu X, Zhang R, Sun L, Gan N, Wang A. Ursodeoxycholic acid stimulates alveolar fluid clearance in LPS-induced pulmonary edema via ALX/cAMP/PI3K pathway. J Cell Physiol. 2019;234(11):20057-65. Epub 2019/04/12. doi: 10.1002/jcp.28602

For the present invention, the respiratory tract infection includes infections caused by any virus selected from the group consisting of coronaviruses, influenza viruses, rhinoviruses, RS viruses, parainfluenza viruses, and adenoviruses. In one embodiment, the composition is used for coronavirus infection among respiratory tract infections. The coronavirus infection is caused by any virus selected from the group consisting of HCoV-229E, HCoV-OC43, HCoV-NL63, HCoV-HKU1, severe acute respiratory syndrome coronavirus (SARS-CoV), middle east respiratory syndrome coronavirus (MERS-CoV), and novel coronavirus (SARS-CoV-2). The novel coronavirus infection is referred to as COVID-19. In one preferred embodiment, the composition is used for treating novel coronavirus infection (COVID-19).

For the present invention, treating (treatment of) a disease or condition includes reducing the risk of developing the disease or condition, delaying or preventing the development of the disease or condition, and stopping or delaying the progression of the disease or condition. The therapy includes curative therapy (therapy to remove the cause) and symptomatic therapy (therapy to ameliorate symptoms). Actions for amelioration or treatment include medical actions performed by physicians and nurses, midwives, and others under the direction of physicians, and non-therapeutic actions performed by non-physicians, such as pharmacists, nutritionists (including registered dietitian nutritionists and sports nutritionists), public health nurses, midwives, nurses, clinical laboratory technicians, sports instructors, drug manufacturers, drug sellers, food manufacturers, food sellers, and others. Furthermore, prevention or reduction of the risk of developing disease or condition includes recommendations for intake of specific foods and nutritional guidance (including guidance on nutrition necessary for medical treatment of injured or sick persons and guidance on nutrition for the maintenance and promotion of good health).

### [Composition]

### (Food compositions, etc.)

The composition of the present invention can be in the form of a food composition or pharmaceutical composition. Food and pharmaceutical are not limited to those for humans, and may be those for animals other than human, unless especially indicated. The food may be a common food, functional food, or nutritional composition, or a therapeutic diet (diet for the purpose of therapy, for which a medical practitioner writes a dietary prescription, and which is cooked by a dietitian or the like according to the prescription), dietetic food, ingredient-modified food, care food, or therapy-supporting food, unless especially indicated. The food is not limited to a solid food, but it may be a food in the form of liquid, for example, drink, drinkable preparation, liquid food, or soup, unless especially indicated. Functional food refers to a food that can give a predetermined functionality to a living body, and includes health foods at large, such as foods for specified health uses (abbreviated as "Tokuho" in Japanese, including conditional foods for specified health use), foods with function claims, foods with health claims including foods with nutrient function claims, foods for special dietary uses, supplements (for example, those of various kinds of dosage forms such as tablet, coated tablet, sugar-coated tablet, capsule and solution), and cosmetic food (for example, diet foods). In the present invention, the "functional foods" include health foods to which the health claim based on the food standards of CODEX (JOINT FAO/WHO FOOD STANDARDS PROGRAMME CODEX ALIMENTARIUS COMMISSION) is applied.

### (Subject)

The composition of the present invention is suitable to be administered to subjects for whom it is desirable or necessary to control growth of bacteria of the genus *Collinsella* in the intestinal tract; subjects with a disease or condition that can be ameliorated by controlling growth of bacteria of the genus *Collinsella* in the intestinal tract; those at risk of infection with viruses responsible for respiratory tract infections, those at risk of infection with coronaviruses such as SARS-CoV-2, patients of respiratory tract infection, and patients of coronavirus infection such as COVID-19. Subjects for whom it is desirable or necessary to control growth of bacteria of the genus *Collinsella* in the intestinal tract include those with a low number of bacteria of the genus *Collinsella* in the intestinal tract. For the present invention, the term administration is used in the sense of administering a pharmaceutical product to a subject as well as having a subject ingest a food or other substance other than a pharmaceutical product.

The age of the subject is not particularly limited, and the subject may be, for example, a neonate (within 28 days of birth); an infant (younger than 1 year of age); a young child (1 to 6 years of age); a child (7 years of age or older and younger than 15 years of age); an adult (15 years of age or older); or a person of 65 years of age or older.

### (Administration route etc.)

The composition of the present invention may be administered orally, parenterally, e.g., via a tube (gastrostomy or enterostomy), or intranasally, but oral administration is preferred.

The composition can be administered to the subject repeatedly or continuously over a long period of time. The period of time is not particularly limited, but in order for the effect to be fully recognized, the composition should be administered continuously over a relatively long period of time, e.g., over 3 days or longer, 1 week or longer, 2 weeks or longer, 1 month or longer, 3 months or longer, 6 months or longer, or 1 year or longer.

The composition may be administered routinely, in advance, such as when the risk is high, or when the need arises. The composition may be administered as a meal, before, after, or between meals, or when the disease or condition that is to be ameliorated by the composition occurs.

### (Dose and content)

The dose of the composition of the present invention may be such an amount that the desired effect is exerted. The dose can be appropriately set in consideration of various factors such as the age, weight, and symptoms of the subject.

The daily dose of the composition can be at least 0.1 mg, preferably at least 0.3 mg, more preferably at least 0.6 mg, even more preferably at least 1 mg, in terms of the amount of the active ingredient. The maximum amount of the active ingredient per day may be 10 g or less, 5 g or less, 1 g or less, 100 mg or less, 60 mg or less, 30 mg or less, or 15 mg or less, no matter how the minimum amount is defined. When two or more types of active ingredients are contained in the composition, the amount of active ingredient means the total amount of the active ingredients contained.

Administration may be once a day or multiple times a day, e.g., 2 to 10 times. The dose of the active ingredient per one time may be, for example, 0.01 mg or more, preferably 0.03 mg or more, more preferably 0.06 mg or more, further preferably 0.1 mg or more. The maximum amount of the active ingredient per one time may be 3 g or less, 1 g or less, 100 mg or less, 33 mg or less, 20 mg or less, 10 mg or less, or 5 mg or less, no matter how is the minimum amount is defined.

The content of the active ingredient in the composition may be appropriately determined according to the form of the composition. For example, the content of the active ingredient based on solid content of the composition can be 0.1% or more, preferably 0.3% or more, more preferably 0.6% or more, further preferably 1% or more. The maximum amount of the active ingredient based on solid content may be 50% or less, 30% or less, 20% or less, 10% or less, or 5% or less, no matter how the minimum amount is defined. For the present invention, % means mass percent unless otherwise stated.

When the composition is a liquid, the content of the active ingredient can be, for example, 0.01% or more, preferably 0.03% or more, more preferably 0.06% or more, further preferably 0.1% or more. The maximum content of the active ingredient when the composition is a liquid may be 5% or less, 3% or less, 2% or less, 1% or less, or 0.5% or less, no matter how the minimum content is defined.

### (Other ingredients and additives)

The composition of the present invention may contain other active ingredients or nutritional components that are acceptable as food or pharmaceutical. Examples of such ingredients include lipids (e.g., milk fat, vegetable fats, fats containing medium-chain fatty acids), proteins (e.g., milk proteins, milk protein concentrate (MPC), whey protein concentrate (WPC), whey protein isolate (WPI), α-lactalbumin (α-La), β-lactoglobulin (β-Lg), heat-denatured whey proteins and enzyme-treated whey proteins, amino acids (e.g., lysine, arginine, glycine, alanine, glutamic acid, leucine, isoleucine, and valine), carbohydrates other than human milk oligosaccharides and constituent sugars of human milk oligosaccharides (e.g., glucose, sucrose, fructose, maltose, trehalose, erythritol, maltitol, palatinose, xylitol, and dextrin), electrolytes (e.g., sodium, potassium, calcium, and magnesium salts), vitamins (e.g., vitamin A, vitamin B1, vitamin B2, vitamin B6, vitamin B12, vitamin C, vitamin D, vitamin E, vitamin K, biotin, folic acid, pantothenic acid, and nicotinic acids), minerals (e.g., copper, zinc, iron, cobalt, and manganese), antibiotics, dietary fibers, and so forth.

The composition of the present invention may contain prebiotics other than human milk oligosaccharides. Prebiotics are indigestible food ingredients that selectively alter growth and activity of certain bacteria in the large intestine, thereby favorably affecting the host and improving the host's health. One or more types of prebiotics other than human milk oligosaccharides may be used in the composition.

Prebiotics other than human milk oligosaccharides are not particularly limited so long as they do not interfere with the effect of the active ingredient contained in the composition. Examples of prebiotics other than human milk oligosaccharides include galactooligosaccharides, fructooligosaccharides, xylooligosaccharides, isomaltooligosaccharides, raffinose, lactulose, lactosucrose, soy oligosaccharides, coffee oligosaccharides, dietary fibers, and gluconic acid.

The composition may also further contain an additive acceptable for foods or pharmaceuticals. Examples of such an additive include inactive carriers (solid and liquid carriers), excipients, surfactants, binders, disintegrating agents, lubricants, dissolving aids, suspending agents, coating agents, colorants, preservatives, buffering agents, pH adjustors, emulsifiers, stabilizers, sweeteners, antioxidants, perfumes, acidulants, and natural substances. More specific examples include water, other aqueous solvents, pharmaceutically acceptable organic solvents, collagen, polyvinyl alcohol, polyvinylpyrrolidone, carboxyvinyl polymer, sodium alginate, water-soluble dextran, water-soluble dextrin, carboxymethyl starch sodium, pectin, xanthan gum, gum arabic, casein, gelatin, agar, glycerin, propylene glycol, polyethylene glycol, vaseline, paraffin, stearyl alcohol, stearic acid, human serum albumin, mannitol, sorbitol, lactose, sucralose, stevia, aspartame, acesulfame potassium, citric acid, lactic acid, malic acid, tartaric acid, phosphoric acid, acetic acid, fruit juice, vegetable juice, and so forth.

### (Dosage form or shape)

The composition of the present invention in the form of a food may be prepared in an arbitrary form such as solid, liquid, mixture, suspension, powder, granule, paste, jelly, gel, and capsule. The composition of the present invention in the form of a food can be made in such an arbitrary form as dairy product, supplement, confectionery, drink, drinkable preparation, seasoning, processed food, daily dish, and soup. More specifically, the composition of the present invention may be in the form of liquid diet, semi-liquid diet, jelly, gel, powder, special formula powdered milk, special formula liquid milk, powdered and liquid milk for pregnant and nursing women, fermented milk, bar, mousse, chocolate, biscuit, ice cream, fermented milk, lactic acid bacteria beverage, lactic beverage, milk beverage, soft drink, tablet, cheese, bread, biscuit, cracker, pizza crust, food for sick persons, nutritional food, frozen food, processed food, or the like, or may be in a form of granule, powder, paste, thick solution or the like for being mixed in drink or food and then ingested. Granules and powders can be in the form of cubes or sticks (single-dose packets). For the present invention, special formula powdered milk means a product obtained from a product produced by processing a food made from raw milk, cow's milk, special cow's milk, or raw water buffalo's milk or a food made by using them as a main raw material, by adding nutrients necessary for infants and young children, and making the resulting composition into powder as defined in the "Ministerial Ordinance on Milk and Milk products Concerning Compositional Standards, etc. (hereinafter referred to as the "Ministerial Ordinance on Milk, etc.")". For the present invention, special formula liquid milk means a product obtained from a product produced by processing a food made from raw milk, cow's milk, special cow's milk, or raw water buffalo's milk or a food made by using them as a main raw material, by adding nutrients necessary for infants and young children, and making the resulting composition into liquid as defined in the Ministerial Ordinance on Milk, etc.

Special formula powdered milk and special formula liquid milk (these are sometimes collectively referred to as special formula powdered milk etc., and the explanation of the special formula powdered milk etc. may be made for special formula powdered milk as an example, but such an explanation shall also apply to special formula liquid milk) can be for infants, follow-up (as a supplement to weaning food), low birth weight infants, children, adults, expectant mothers, nursing mothers, elderly persons, those with allergies, those with lactose intolerance, and those with congenital metabolic disorders. Preferred examples of special formula powdered milk etc. are those for infants, follow-up, low birth weight infants, and children.

The composition of the present invention as a pharmaceutical can be in an arbitrary dosage form, for example, those suitable for oral administration including solid preparations such as tablet, granule, powder, pill, and capsule and liquid preparations such as solution, suspension, and syrup, gel, aerosol, or the like.

### (Others)

In the manufacture of the composition of the present invention, time of adding the active ingredient can be appropriately chosen. Unless the characteristics of the active ingredient are not markedly degraded, the time of the addition is not particularly limited. For example, the active ingredient can be blended by mixing it with raw materials. Alternatively, the active ingredient can be added at the final stage of the manufacture to produce a composition containing the active ingredient.

The composition of the invention can have an indication describing the purpose of use (intended use), or an indication describing that administration of the composition is recommended for specific subjects.

The composition of the present invention can have an indication describing that, with the composition or the active ingredient, growth of bacteria of the genus *Collinsella* in the intestinal tract can be controlled, growth of bacteria of the genus *Collinsella* in the intestinal tract can be promoted, a disease or condition that can be ameliorated by controlling (preferably promoting) growth of bacteria of the genus *Collinsella* can be treated, the composition can be used as a prebiotic or can be used as a synbiotic (a combination of a probiotic and a prebiotic), or related diseases can be treated with the composition, or respiratory tract infections such as coronavirus infections can be treated with the composition. The indication may be a direct or indirect indication. Examples of the direct indication include descriptions on tangible articles such as the product itself, package, container, label, and tag, and examples of the indirect indication includes advertising and campaign activities using such places or means as web site, shop, pamphlet, exhibition, seminar such as media seminar, book, newspaper, magazine, television, radio, postal matter, E-mail, and sound.

Hereafter, the present invention will be more specifically explained with reference to examples. However, the technical scope of the present invention is not limited by these examples.

### Examples

### [Preparation of sugar solutions]

2'-FL (Jennewein Biotechnologie GmbH), 3'-SL (Carbosynth), 6'-SL (Tokyo Kasei Kogyo, #S0886), lactose (Fujifilm Wako Pure Chemicals Corporation, #128-00095), and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 70 µL was added to 7 mL of GAM semisolid medium without dextrose (Nissui, #05460) for glycolysis test and incubated at 37°C for 24 hours under anaerobic conditions. Each fecal culture was added to 6 wells in total of a 96-well deep well plate in a volume of 900 µL each. To the 6 wells, the sugar solutions (5 types) and sterilized water were added, respectively, in a volume of 100 µL each (final concentrations of the sugar solutions, 1 mass %). After incubation at 37°C for 24 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

The results are shown in Fig. 1. Compared with the sterile water group as the control, the occupancy rate of bacteria of the genus *Collinsella* significantly increased or tended to increase in the groups in which 2'-FL, 3'-SL, 6'-SL, lactose, and Neu5Ac were added. These results suggested that 2'-FL, 3'-SL, 6'-SL, lactose, and Neu5Ac promote growth of bacteria of the genus *Collinsella* in the human intestinal tract. By the way, bacteria of the genus *Bifidobacterium* were detected in all the thawed fecal solutions.

In this example, a growth-promoting effect of lactose for bacteria of the genus *Collinsella* was confirmed. HMOs contain lactose as a constituent sugar, and HMOs are thought to generate lactose in the human digestive tract after ingestion (Non-patent document 6). Therefore, it is considered that a growth-promoting effect for bacteria of the genus *Collinsella* can be obtained with HMOs.

In this example, a growth-promoting effect of Neu5Ac for bacteria of the genus *Collinsella* was confirmed. HMOs containing Neu5Ac as a constituent sugar are thought to generate Neu5Ac in the human digestive tract after ingestion (Non-patent documents 6 to 8). Therefore, it is considered that a growth-promoting effect for bacteria of the genus *Collinsella* can be obtained with HMOs containing Neu5Ac as a constituent sugar (sialylated HMOs and HMOs containing sialic acid).

In this example, a growth-promoting effect for bacteria of the genus *Collinsella* was confirmed for all of 2'-FL, 3'-SL, and 6'-SL. Therefore, it is considered that a growth-promoting effect for bacteria of the genus *Collinsella* can be obtained with fucosylated HMOs and sialylated HMOs.

### [References cited in the examples]

Non-patent document 6: Masi AC, Stewart CJ. Untangling human milk oligosaccharides and infant gut microbiome. iScience. 2021;25(1):103542. Published 2021 Dec 1. doi:10.1016/j.isci.2021.103542
Non-patent document 7: Nishiyama K, Yamamoto Y, Sugiyama M, et al. Bifidobacterium bifidum Extracellular Sialidase Enhances Adhesion to the Mucosal Surface and Supports Carbohydrate Assimilation. mBio. 2017;8(5):e00928-17. Published 2017 Oct 3. doi:10.1128/mBio.00928-17
Non-patent document 8: Kiyohara M, Tanigawa K, Chaiwangsri T, Katayama T, Ashida H, Yamamoto K. An exo-alpha-sialidase from bifidobacteria involved in the degradation of sialyloligosaccharides in human milk and intestinal glycoconjugates. Glycobiology. 2011;21(4):437-447. doi:10.1093/glycob/cwq175

## Claims

1. A composition for controlling growth of bacteria of the genus *Collinsella* in the intestinal bacterial microbiota, which contains any selected from the group consisting of a human milk oligosaccharide and a constituent sugar of a human milk oligosaccharide.

2. The composition according to claim 1, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid.

3. The composition according to claim 2, which contains any selected from the group consisting of 2'-fucosyllactose, 3'-sialyllactose, 6'-sialyllactose, lactose, and N-acetylneuraminic acid.

4. The composition according to any one of claims 1 to 3, which is for use as a prebiotic or synbiotic.

5. A composition for treating a disease or condition that can be ameliorated by controlling growth of bacteria of the genus *Collinsella* in the intestinal tract, which contains any selected from the group consisting of a human milk oligosaccharide and a constituent sugar of a human milk oligosaccharide.

6. The composition according to claim 5, wherein the disease or condition that can be ameliorated by controlling growth of bacteria of the genus *Collinsella* in the intestinal tract is a respiratory tract infection.

7. A composition for treating a respiratory tract infection, which contains any selected from the group consisting of a human milk oligosaccharide and a constituent sugar of a human milk oligosaccharide.

8. The composition according to claim 6 or 7, wherein the respiratory tract infection is a coronavirus infection.

9. The composition according to claim 6 or 7, wherein the respiratory tract infection is a novel coronavirus infection (COVID-19).

10. The composition according to any one of claims 1 to 6, wherein the control of growth is promotion of growth.
